# EUROPEAN PATENT SPECIFICATION

(11) **EP 3 326 661 B1**
(45) Date of publication and mention of the grant of the patent: **18.11.2020**
(21) Application number: 16828086.5
(22) Date of filing: 21.07.2016
(51) Int. Cl.: B33Y 70/00, A61L 27/26, A61L 27/38, A61L 27/50, A61L 27/54, C09D 11/00, B29C 67/02

(54) **BIO-INK COMPOSITION HAVING IMPROVED PHYSICAL AND BIOLOGICAL PROPERTIES**
BIOTINTENZUSAMMENSETZUNG MIT VERBESSERTEN PHYSIKALISCHEN UND BIOLOGISCHEN EIGENSCHAFTEN
COMPOSITION DE BIO-ENCRE AYANT DES PROPRIÉTÉS PHYSIQUES ET BIOLOGIQUES AMÉLIORÉES

(30) Priority: 21.07.2015 KR 20150103313
(43) Date of publication of application: 30.05.2018
(73) Proprietor: BioinkSolution Inc., 807, Hoguk-ro, Buk-gu Daegu 41404 (KR)
(72) Inventor: HWANG, Mi Sun, Daegu 42129 (KR)
(74) Representative: Isarpatent
(86) International application number: PCT/KR2016/007962
(87) International publication number: WO 2017/014582

(56) References cited:
- WO-A1-2013/158508
- WO-A2-2007/124023
- US-A1- 2011 150 823
- US-A1- 2012 089 238
- US-A1- 2015 057 786
- US-B2- 6 773 713
- FERRIS, C. J. ET AL.: 'Bio-ink for On-demand Printing of Living Cells' BIOMATERIALS SCIENCE vol. 1, 2013, pages 224 - 230, XP055348567
- MELCHELS, F. P. W. ET AL.: 'Development and Characterisation of a New Biolink for Additive Tissue Manufacturing' JOURNAL OF MATERIALS CHEMISTRY B vol. 2, 2014, pages 2282 - 2289, XP055348585
- GU QI ET AL: "Three-dimensional bio-printing", SCIENCE CHINA LIFE SCIENCES, ZHONGGUO KEXUE ZAZHISHE, CHINA, vol. 58, no. 5, 15 April 2015 (2015-04-15) , pages 411-419, XP035939640, ISSN: 1674-7305, DOI: 10.1007/S11427-015-4850-3 [retrieved on 2015-04-15]

<110> Bioink solutions Inc.
<120> BIO-INK COMPOSITION HAVING IMPROVED PHYSICAL AND BIOLOGICAL
   PROPERTIES
<130> P45657-WOEP
<140> 16828086.5
   <141> 2016-07-21
<150> 10-2015-0103313
   <151> 2015-07-21
<160> 4
<170> KoPatentIn 3.0
<210> 1
   <211> 20
   <212> PRT
   <213> Artificial Sequence
<220>
   <223> BMP mimicking peptide
<400> 1
   Lys Ile Pro Lys Ala Ser Ser Val Pro Thr Glu Leu Ser Ala Ile Ser
     1               5                  10                  15
   Thr Leu Tyr Leu
               20
<210> 2
   <211> 7
   <212> PRT
   <213> Artificial Sequence
<220>
   <223> TGF mimicking peptide 1
<400> 2
   Ala Asn Val Ala Glu Asn Ala
    1               5
<210> 3
   <211> 6
   <212> PRT
   <213> Artificial Sequence
<220>
   <223> TGF mimicking peptide 2
<400> 3
   Leu Ile Ala Asn Ala Lys
    1               5
<210> 4
   <211> 15
   <212> PRT
   <213> Artificial Sequence
<220>
   <223> VEGF mimicking peptide
<400> 4
   Lys Leu Thr Trp Gln Glu Leu Tyr Gln Leu Lys Tyr Lys Gly Ile
    1               5                  10                  15

## Description

### THECHNICAL FIELD

The present invention relates to a bio-ink composition having improved physical and biological properties. More particularly, the present invention relates to a bio-ink composition having cell compatibility, high viscosity which is easy to print, a strong shear-thinning tendency, formation of fast crosslinking, and suitable mechanical properties after printing.

### BACKGROUND OF THE INVENTION

Three-dimensional cell culture technology has developed into a technology capable of producing cells and tissues in an environment similar to a living tissue *in vitro* and has been applied to various research fields related to cell growth and differentiation, and tissue & organ formation. Such tissue-like organs may be useful in the study of toxicity and pharmacokinetics of drugs in place of actual tissues or organs and thus may reduce direct experimental applications to human specimens and other mammals. It also contributes to tissue and organ engineering design as an important element of tissue engineering techniques that are intended to replace or treat damaged tissue and organs.

Three-dimensional bio-printing technology has become a useful device for precisely manufacturing such tissue-like organs and implantable structures. This technique makes it possible to create micro- and macro-organizational structures that almost mimic the actual human tissues. However, bio-ink, a biomaterial that carries living cells during bio-printing, has many limitations in its utilization. The bio-ink to be applied to bio-printing is required to have excellent bio-compatibility , to have a physical property capable of smoothly passing through a dispensing nozzle of a micro-aperture to print a desired pattern, and to maintain the role of a mechanical support while providing a cell-specific signal after printing, and the like. Although natural or synthetic hydrogel bio-inks have been developed and are currently in use in the field of three-dimensional bio-printing, bio-inks based on conventional hydrogels show considerable limitations in physical and biological aspects such as biocompatibility, printing suitability, geometrical precision and the degree of precision.

WO-A-2007124023 describes a ink-jet printing of a three dimensional construct.

US-A-2011150823 describes a cell tissue gel comprising collagen and hyaluronan at a weight ratio of 0.01-100:1.

WO-A-2013158508 describes a bio-ink comprising multiple cells incorporated into a UV cross-linkable material.

US-A-2012089238 describes a three dimensional printing of a bio-ink composition.

Gu Qi et al. (Science China Life Sciences, 2015, vol. 58, no. 5, pages 411-419) describes a use of gelatin methacrylate in combination with a lubricant and a further cell carrier material.

### DETAILED DESCRIPTION OF THE INVENTION

### TECHNICAL PROBLEM

Accordingly, the present inventors have completed the present invention after they have found that the above problems can be solved by bio-ink compositions comprising specific amounts of cellular carrier materials, viscosity enhancers, lubricants and structuring materials, wherein the composition possesses high viscosity, strong shear-thinning tendency, fast crosslinking and mechanical properties to maintain the printed structure. In addition, a tissue-derived extracellular matrix component may be added to the bio-ink composition to have tissue specificity or to induce differentiation into a specific tissue by adding a specific cell differentiation regulator.

Accordingly, an aspect of the present invention is to provide bio-ink composition comprising 0.05-60 x 10⁶/mL of cell, 0.1 to 10 w/v % of gelatin as a cell carrier material, 0.01 to 1 w/v % of hyaluronic acid as a viscosity enhancer, 1 to 30 v/v % of glycerol as a lubricant and 0.1 to 10 w/v % of a structural material, wherein the structural material is gelatin methacrylate .

Another aspect of the present invention is to provide a method for preparing the bio-ink composition, the method comprising: (a) charging a bio-ink composition of the present invention into a three-dimensional bio-printer; (b) three-dimensionally printing a desired tissue-like organ; and (c) cross-linking the three-dimensionally printed bio-ink composition.

Described and not claimed *per se* is a tissue-like organ produced according to the method.

### TECHNICAL SOLUTION

An embodiment according to an aspect of the present invention provides a bio-ink composition comprising 0.05-60 x 10⁶/mL of cell, 0.1 to 10 w/v % of gelatin as a cell carrier material, 0.01 to 1 w/v % of hyaluronic acid as a viscosity enhancer, 1 to 30 v/v % of glycerol as a lubricant and 0.1 to 10 w/v % of a structural material, wherein the structural material is gelatin methacrylate.

Another embodiment according to an aspect of the present invention provides a method for preparing the bio-ink composition, the method comprising: (a) charging a bio-ink composition of the present invention into a three-dimensional bio-printer; (b) three-dimensionally printing a desired tissue-like organs; and (c) cross-linking the three-dimensionally printed bio-ink composition.

Described and not claimed *per se* is a tissue-like organ produced according to the above method.

Hereinafter, the present invention will be described in detail.

The present invention provides a bio-ink composition comprising 0.05-60 x 10⁶/mL of cell, 0.1 to 10 w/v % of gelatin as a cell carrier material, 0.01 to 1 w/v % of hyaluronic acid as a viscosity enhancer, 1 to 30 v/v % of glycerol as a lubricant and 0.1 to 10 w/v % of a structural material, wherein the structural material is gelatin methacrylate.

In the present invention, the cell may be preferably selected from the group consisting of a stem cell, an osteoblast, a myoblast, a tenocyte, a neuroblast, a fibroblast, a glioblast, a germ cell, hepatocyte, a renal cell, a sertoli cell, a chondrocyte, an epithelial cell, a cardiovascular cell, a keratinocyte, a smooth muscle cell, a cardiomyocyte, glial cell, a endothelial cell, a hormone-secreting cell, a immune cell, a pancreatic islet cell and a neuron, but is not limited thereto.

The cells used in the engineered tissue of the present invention may be cultured in any manner known in the art. Cell and tissue culture methods are well known in the art and are described, for example, in Cell & Tissue Culture: Laboratory Procedures: Freshney (1987), Culture of Animal Cells: A Manual of Basic Techniques. General mammalian cell culture techniques, cell lines, and cell culture systems that can be used for the present invention are also described in Doyle, A., Griffiths, JB, Newell, DG, (eds.) Cell and Tissue Culture: Laboratory Procedures. Wiley (1998).

The cells may also be cultured with a cell differentiation agent that induces differentiation of cells according to a desired cell line. For example, stem cells are incubated in contact with differentiation media to produce a range of cell types. Multiple types of differentiation media are suitable. The stem cells may be incubated in contact with a differentiation medium including, but not limited to, osteogenic differentiation medium, chondrogenic differentiation medium, adipogenic differentiation medium, neural differentiation medium, myocardial cell differentiation medium, and intestinal cell differentiation medium (e.g., epidermis of the intestine).

Additionally, the cells may be cultured with growth factors, cytokines and the like. "Growth factor" refers to a protein, polypeptide, or polypeptide complex which is produced by a cell and capable of affecting itself and/or various other adjacent or distant cells, including a cytokine. Growth factors typically affect the growth and/or differentiation of certain types of cells either genetically or in response to a number of biochemical or environmental stimuli. Some, but not all, of the growth factors are hormones. Exemplary growth factors include insulin, insulin-like growth factor (IGF), nerve growth factor (NGF), vascular endothelial growth factor (VEGF), keratinocyte growth factor (KGF), fibroblast growth factor (FGF) including basic FGF (bFGF), platelet-derived growth factor (PDGF) including PDGF-AA and PDGF-AB, bone morphogenetic protein (BMP) including BMP-2 and BMP-7, hepatocyte growth factor (HGF), transforming growth factor alpha (TGF-a), transforming growth factor Beta (TGF- β) including TGFβ1 and TGFβ3, Epidermal growth factor (EGF), granulocyte-macrophage colony-stimulating factor (GM-CSF), granulocyte colony-stimulating factor (G-CSF), interleukin--6(IL-6), and IL-8. Growth factors have been described in Molecular Cell Biology, Scientific American Books, Darnell et al., Eds., 1986; Princeplengs of Tissue Engineering, 2d ed., Lanza et al., Eds., Academic Press, 2000. Those skilled in the art are able to understand that any culture in the conditioned media as described herein and all culture-derived growth factors are within the scope of the present invention.

In the bio-ink composition of the present invention, the cell carrier material includes an amount of 0.1-10 w/v %, 0.5-10 w/v %, 1-10 w/v %, 1.5-10 w/v %, 2-10 w/v %, 0.1-8 w/v %, 0.5-8 w/v %, 1-8 w/v %, 2-8 w/v %, 0.1-6 w/v %, 0.5-6 w/v %, 1-6 w/v %, 2-6 w/v %, 0.1-5 w/v %, 0.5-5 w/v %, 1-5 w/v % or 2-5 w/v %.

In the bio-ink composition of the present invention, the viscous enhancer includes an amount of 0.01-1 w/v %, 0.05-1 w/v %, 0.1-1 w/v %, 0.01-0.8 w/v %, 0.05-0.8 w/v % or 0.1-0.8 w/v %.

In the bio-ink composition of the present invention, the lubricant includes an amount of 1-30 v/v %, 0.1-20 v/v %, 0.5-20 v/v %, 1-20 v/v %, 0.1-15 v/v %, 0.5-15 v/v % or 1-15 v/v %.

In the bio-ink composition of the present invention, the structural material includes an amount of 0.1-10 w/v %, 0.5-10 w/v %, 1-10 w/v %, 0.1-8 w/v %, 0.5-8 w/v %, 1-8 w/v %, 0.1-5 w/v %, 0.5-5 w/v % or 1-5 w/v %.

The inventors of the present invention have tested the physical and biological properties of compositions having various combinations of components in order to develop a bio-ink composition exhibiting physical and biological properties suitable for its application to three-dimensional bio-printing. As a result, the bio-ink composition comprising the above described combinations of components and their ratios exhibits high viscosity, strong shear-thinning tendency, fast crosslinking and suitable mechanical properties after printing. Thus, it has been found that the bio-ink composition as described above is particularly suitable for three-dimensional bio-printing for the production of tissue-like organs.

In the bio-ink composition of the present invention, the cell carrier material is gelatin. Described and not claimed *per se* is that the cell carrier material may be selected from ones suitable for preparing a uniformed cell suspension and exhibiting excellent printing tendency, while it may be one or more selected from the group consisting of collagen, alginate, agar, agarose, pluronic and polyvinyl alcohol, but is not limited thereto.

In the bio-ink composition of the present invention, the viscous enhancer is hyaluronic acid. Described and not claimed *per se* is that the viscous enhancer may be selected from ones suitable for maintaining excellent printing tendency and the initial strength of the bio-ink, while it may include dextran, but is not limited thereto.

In the bio-ink composition of the present invention, the lubricant is glycerol. Described and not claimed *per se* is that the lubricant may be selected from other materials capable of minimizing shear rate and improving dispensing speed.

In the bio-ink composition of the present invention, the structural material is gelatin methacrylate. Described and not claimed per se is that the structural material may be selected from other materials capable of forming rapid crosslinking and maintaining mechanical strength, and its non-limiting examples may be one or more selected from the group consisting of fibrinogen, hyaluronic acid modified to be chemically crosslinkable (e.g., methacrylated hyaluronic acid, and thiolated hyaluronic acid, etc.), gelatin modified to be chemically crosslinkable (e.g., thiolated gelatin, etc.), collagen, alginate, methylcellulose, chitosan, chitin, synthetic peptides, and polyethylene glycol-based hydrogels.

In the present invention, the gelatin serves as the cell carrier material since it exhibits temperature-sensitive properties. That is, gelatin is liquefied at 37 ° C and has a characteristic of solidifying at room temperature or below. Described and not claimed per se is that the fibrinogen is not only suitable as a structural material in terms of stability of the gel, but also can be selected as a preferable structural material in that it forms a microenvironment suitable for attachment and differentiation of cells after the cells are printed. In the present invention, hyaluronic acid and glycerol can be suitably used in the composition of the present invention in terms of dispensing uniformity and prevention of clogging of the nozzle when three-dimensional bio-printing is applied.

Specifically, according to one Example of the present invention, it was confirmed that the dispensing rate of the composition according to the present invention is various depending on the content of gelatin contained in the bio-ink composition. The lower the dispensing rate, the greater the resolution of the pattern, but it was confirmed that the resolution of the pattern was increased as the gelatin was more than 35 mg/mL. On the other hand, hyaluronic acid in various concentrations was included in the bio-ink composition and the properties thereof were evaluated. As a result, it was confirmed that hyaluronic acid has an effect of significantly improving the uniformity of the pattern although it does not affect the dispensing rate of the composition (Example 3).

Thus, in the bio-ink composition of the present invention, it can be known that gelatin has a high contribution in terms of resolution of pattern, while hyaluronic acid in terms of uniformity of pattern.

In the present invention, cells are bio-printed by depositing or extruding bio-ink from a three-dimensional bio-printer. The bio-ink of the present invention comprises a liquid, semi-solid, or solid composition comprising a plurality of cells. Bio-inks comprise liquid or semi-solid cell solutions, cell suspensions, or cell concentrates. The bio-ink composition comprises: 1) preparing a bio-ink by mixing a plurality of cells or cell aggregates with a biocompatible liquid or gel at a predetermined ratio, and 2) preparing a bio-ink having a desired cell density and viscosity by densifying the bio-ink. Specifically, the densification of the bio-ink is realized by centrifugation, tangential flow filtration ("TFF"), or a combination thereof, and densification of the bio-ink forms an extrudable composition to form multicellular aggregates or multicellular bodies. The term "extrudable" means that it can be formed by passing (for example, under pressure) through a nozzle or orifice (e.g., one or more holes or tubes). Densification of the bio-ink is also derived from growing the cells at an appropriate density. The cell density required for the bio-ink depends on the cells to be used and on the tissue or organ to be produced.

The present invention also provides a bio-ink composition, wherein the bio-ink composition further comprises a tissue-derived component.

The tissue-derived component means one in which a specific tissue of an animal such as cartilage, kidney, heart, liver, and muscle is de-cellularized, while a material mainly composed of an extracellular matrix being gelified. This tissue-derived component may be included to enhance the tissue specificity of the bio-ink composition.

In order for the cells contained in the bio-ink composition to normally grow and differentiate in a three-dimensional printed tissue-like organ, it is necessary to create a natural environment similar to the microenvironment of the original tissue in the body. It is practically impossible for any artificially generated composition to reproduce all the properties of natural tissue-derived components such as an extracellular matrix. Thus, by adding de-cellularized tissue-derived components to the bio-ink composition, it is possible to create a micro-environment in which cells can normally grow and differentiate.

Since the de-cellularized tissue-derived component has individual tissue-specific constituents and phases formed through interaction with cells in the human body, it can provide an environment favorable for cell growth and differentiation. Therefore, by adding tissue-derived components to the bio-ink composition of the present invention, it is possible to keep the cells in their normal form and to favorably preserve their functions.

The tissue from which the tissue-derived component of the present invention can be obtained is not particularly limited and is selected according to the tissue-like organs to be prepared. It can be used after de-cellularization and gelification. Methods for de-cellularization and gelification of tissues are well known in the art and can be easily selected by those skilled in the art to which the present invention belongs.

According to one Example of the present invention, muscle cells were printed using a bio-ink composition containing components derived from muscle tissue. As a result, it was confirmed that the proliferation and differentiation of muscle cells were improved as compared with the composition containing no tissue-derived component.

The present invention also provides a bio-ink composition which further comprises a regulator material of differentiation.

The regulator material of differentiation may be included to induce the differentiation of stem cells (including embryonic stem cells, inducible pluripotent stem cells, and adult stem cells) contained in the bio-ink composition into specific cells. Its non-limiting examples include such chemicals as bone morphogenetic protein-2 (BMP-2) when inducing differentiation of bio-printed stem cells into bone cells, growth factors such as TGF-beta3 when inducing differentiation into chondrocytes, dexamethasone when inducing differentiation into osteocytes, and 5-azacytidine (5-azacytidine) when inducing differentiation into muscle cells. In addition, the bio-ink composition of the present invention may further comprise cytokines, peptides, or low-molecular chemicals. In this way, the bio-ink composition of the present invention may comprises growth factors, cytokines, peptides, low-molecular chemicals, or the like capable of inducing differentiation of stem cells into specific tissue cells. Thus, it can be utilized as a functional bio-ink capable of inducing the tissue-like structure including the prepared stem cells to be differentiated into a specific tissue after bio-printing.

In the present invention, the bio-ink composition may further comprise a cell culture medium. The cell culture medium is meant to include any medium suitable for a desired cell. Its non-limiting examples include Dulbecco's phosphate buffered saline, Earle's balanced salts, Hank's balanced salts, Tyrode's salts, Alsever's solution, Gey's balanced salt solution, Kreb's-Henseleit buffer modified, Kreb's-ringer Bicarbonate Buffer, Puck's saline, Dulbecco's Modified Eagle's medium (DMEM), Dulbecco's Modified Eagle's medium (DMEM)/ Nutrient Mixture F-12 Ham, nutrient mixture F-10 ham (Ham's F-10), medium 199, Eagle's minimal essential medium (EMEM), RPMI 1640 Medium, Ames' Media, BGJb medium (Fitton-Jackson modification), Click's medium, CMRL-1066 medium, Fischer's medium, Glascow Minimum Essential Medium (GMEM), Iscove's Modified Dulbecco's Medium (IMDM), L-15 medium (Leibovitz), McCoy's 5A Modified medium, NCTC medium, Swim's S-77 medium, Waymouth medium, William's E Medium, or combinations thereof, but are not limited thereto. In addition, the cell culture medium may further contain albumin, selenium, transferrin, fetuin, sugars, amino acids, vitamins, growth factors, cytokines, hormones, antibiotics, lipids, lipid carriers, and cyclodextrins or combinations thereof.

In the present invention, the bio-ink composition may further comprise a substance for promoting cell adhesion and/or an antioxidant.

In addition, the bio-ink composition of the present invention may further comprise a substance that inhibits cell death (e.g., necrosis, apoptosis, or autonomic absorption). Non-limiting examples of such a substance may be selected from small molecules, antibodies, peptides, peptibodies, anti-TNF substances, substances inhibiting the activity of interleukins, substances inhibiting the activity of interferons, substances inhibiting the activity of GCSF (granulocyte colony-stimulating factor), substances inhibiting the activity of macrophage inflammatory protein, substances inhibiting the activity of TGF-B (transformation growth factor B), substances inhibiting the activity of MMP (matrix metalloproteinase), substances inhibiting the activity of the MAPK/JNK signaling cascade, substances inhibiting the activity of Src kinase, a substance inhibiting the activity of JAK (Janus kinase), or combinations thereof.

The present invention also provides a bio-ink composition which further comprises a thrombin solution or a photoinitiator.

The present invention also provides a method for preparing a tissue-like organ, the method comprising: (a) charging a bio-ink composition of the present invention into a three-dimensional bio-printer; (b) three-dimensionally printing a desired tissue-like organ; and (c) cross-linking the three-dimensionally printed bio-ink composition.

The bio-ink composition provided in the present invention is easy to prepare various tissue-like organs. Bio-printing technology has been developed in the art as a useful tool for preparing tissue-like organs. On the other hand, conventional bio-ink compositions for use in bio-printing have a problem in that it is difficult to have sufficient physical rigidity because of its liquid form, or excessive physical rigidity, so that the cells cannot be sufficiently viable during the printing process. The bio-ink composition of the present invention is applied to three-dimensional bio-printing to prepare tissue-like organs, with its advantages of maintaining a sufficient physical rigidity and sufficiently ensuring the survival of the cells in the case of being sprayed by the bio printer. Thus, it exhibits physical and biological properties that are very suitable for the preparation of tissue-like organs.

According to another Example of the present invention, the bio-ink composition of the present invention was applied to a bio-printer and then cultured for one week after bio-printing. As a result, it was observed that the survival rate of cells was maintained at 80% or more. Thus, it was verified that the cell death was minimized in the process of extrusion and injection by the nozzle and the micro-environment favorable for cell survival was provided sufficiently.

In addition, after observing that the shape and size of the tissue structure prepared by the bio-ink composition of the present invention maintained well after culturing for 21 days in the culture medium, it was verified that it has a physical rigidity suitable for preparing a tissue-like organ or tissue structure.

The bio-ink composition in the present invention enables the lamination of three-dimensional structures. The bio-ink composition of the present invention containing a sufficient amount of cells can be used to form tissue-like organs by being laminated by a three-dimensional bio-printer.

The term "bio-printing" as used herein refers to the use of three-dimensional precise cell depositions (e.g., cell solutions, cell-containing gels, cell suspensions, cell concentrates, multicellular aggregates, multicellular bodies, etc.) by using a methodology compatible with automated, computer-assisted, and three-dimensional prototyping devices (e.g., bio-printers).

The bio-printing method in the present invention is continuous and/or substantially continuous. A non-limiting example of a continuous bio-printing method is to spray bio-ink from a bio-printer through a dispense tip (e.g., a syringe, capillary, etc.) connected to a reservoir of bio-ink. The continuous bio-printing method is to spray the bio-ink in the repeating pattern of the functional unit. The repeating functional unit has any suitable geometry including, for example, circular, square, rectangular, triangular, polygonal, and irregular geometries. In a further embodiment, the repetitive pattern of bio-printed functional units comprises a layer and is bio-printed adjacently to form a tissue or organ fabricated into a plurality of layers. Specifically, it is bio-printed adjacently to form a tissue or organ fabricated into 2, 3, 4, 5, 6, 7, 8, 9, 10, 11, 12, 13, 14, 15 or more layers.

The bio-printed functional units are repeated in a tessellated pattern. The "tessellated pattern" is a planar shape that fills a plane without overlap or gap. The advantages of continuous and/or tessellated bio-printing may include increased productivity of bio-printed tissues. Another non-limiting potential advantage is that it eliminates a need to align bio-printers with previously deposited bio-ink elements. Depending on situations, continuous bio-printing may also render printing a larger tissue from a large storage of bio-ink easier by using a syringe mechanism.

The proper and/or optimal spray distance from the bio-print does not cause the flattening of a material or sticking to the spray needle. The bio-printer spray tip has an internal diameter of about 5, 10, 20, 50, 100, 150, 200, 250, 300, 350, 400, 450, 500, 550, 600, 650, 700, 750, 800, 850, 900, 950, 1000 *µ*m or more, while including its incremental internal diameter within this range. In addition, the bio-ink reservoir of the bio-printer has a capacity of about 0.5, 1, 2, 3, 4, 5, 6, 7, 8, 9, 10, 15, 20, 25, 30, 35, 40, 45, 50, 55, 60, 65, 70, 75, 80, 85, 90, 95, 100 cm² or more, while including its incremental volume within this range. A pump speed may be appropriate and/or optimal when a residual pressure rise in the system is low. A favorable pump speed may depend on the ratio between the cross-sectional area of the reservoir and that of the spray needle, while its higher ratio requires a lower pump speed.

Various types of tissue-like organs can be produced by the methods described above. The pattern or laminate arrangement for laminating the bio-ink composition may be determined by the size, diameter or the like of the tissue-like organs to be prepared. In addition, the number of cells contained in the bio-ink used for preparing the tissue-like organs may be controlled depending on the type of cells, the content of the cell nutrients contained in the bio-ink composition. In addition, according to the above method, the type of cells contained in the bio-ink composition can be variously changed according to the type of tissue-like organs to be prepared. Those skilled in the art will be able to select and utilize appropriate cells according to the type of tissue-like organs to be prepared through three-dimensional bio-printing.

After the bio-ink composition is sprayed and laminated by a three-dimensional bio-printer, the cosslinking of the bio-ink composition may be promoted by the exposure of ultraviolet light or the addition of a cross-linking solution. Such cross-linking allows the laminated bio-ink composition to become a harder structure. On the other hand, the surface of the laminated bio-ink composition may be directly exposed to the ultraviolet light. For instance, it may be exposed to ultraviolet light having a wavelength of 365 nm generated from an 8-watt ultraviolet generator at a distance of 1 to 20 cm for 1 to 1,000 seconds, optionally 20 to 500 seconds or 40 to 240 seconds. Considering such distance and time, those of ordinary skill in the art to which the present invention pertains may understand that a short distance and a strong wavelength can form a sufficient crosslinking even with a short exposure time.

A photoinitiator may be used to facilitate such crosslinking, which means that the photoinitiator acts to induce a rapid crosslinking upon exposure to light. Non-limiting examples of a suitable photoinitiator include acetophenone, benzoin methyl ether, diethoxyacetophenone, benzoylphosphine oxide, and 1-hydroxycyclohexyl phenyl ketone. The amount of the photoinitiator to be added may vary depending on the wavelength and time of the light being exposed. The wavelength of the ultraviolet light for crosslinking may be from 300 to 400 nm, more preferably from 350 to 400 nm, from 350 to 390 nm, from 355 to 380 nm, from 360 to 370 nm, most preferably 365 nm.

Meanwhile, the method of the present invention may further comprise (d) culturing the tissue-like organ in a culture medium. The culture and culture medium are as described above.

Described and not claimed *per se* is a tissue-like organ or implantable tissue structure manufactured according to the method.

Three-dimensional bio-printing technology has the potential to prepare a tissue or organ structure clinically applicable in terms of clinical shape and size. In one Example of the present invention, it has been demonstrated that the bio-ink composition of the present invention may be used to produce complex tissue structures anatomically or functionally applicable. Through the combination of tissue-specific cell types and bio-inks applied to three-dimensional structures, the reproductive ability inherent in the cell can be utilized, thereby leading to the possibility of preparing the desired tissue or organ. In the three-dimensional bio-printed tissue structure, the size of the nozzle allows the integration of cell-containing bio-ink to be finely manipulated in terms of volume and position. In addition, the geometric and constitutive structures of the tissue-like organ may be controlled. Accordingly, the bio-printed tissue structures using the bio-ink composition according to the present invention provide structures that are anatomically and functionally similar to mammalian tissues and organs.

Using the composition of the present invention may not only provide a single structure of a tissue-like organ or implantable tissue, but also manufacture a tissue structure having a significantly improved function in which the linkage among tissues is reproduced via the simultaneous printing of various cells.

In one Example of the present invention, by constructing a tissue structure printed using hepatocytes and vascular cells, a tissue structure printed using osteocytes and chondrocytes, a tissue structure printed using muscle cells and tendon cells, and tissue structure printed using neurons and muscle cells, a structure similar in function to the actual tissue structure in the body was reproduced.

### EFFECTS OF THE INVENTION

Since the bio-ink composition of the present invention exhibits high viscosity, strong shear-thinning tendency, rapid formation of crosslinking and suitable mechanical properties after printing, it may be very useful for the preparation of tissue-like organs using a three-dimensional bio-printing.

### BRIEF DESCRIPTION OF THE DRAWINGS

FIG. 1 is a schematic view showing a process of preparing a three-dimensional hydrogel tissue structure using the bio-ink of the present invention.
FIG. 2 shows the result of evaluating the structural stability of the bio-ink of the present invention after 21 days of culture in the cell culture conditions (A: appearance change of tissue structure after 21 days of culture, B: measurement of tissue structure size change for 21 days after bio-printing).
FIG. 3 shows the change in the stability of the bio-printed structure depending on the content of the viscous enhancer contained in the bio-ink composition.
FIG. 4 is a diagram for evaluating the resolution according to the printing performance and feed rate of the bio ink of the present invention, showing that the pattern resolution is improved as the feed rate is increased and that the uniformity is maintained under the used feed rate.
FIG. 5 shows the result of evaluating the viability of cells for 7 days via fluorescent staining after three-dimensional printing with bio-inks of various compositions containing 3T3 fibroblasts (Gel-MA 50: 50% methacrylated gelatin).
FIG. 6 shows the result of evaluating the viability of cells for 7 days via fluorescent staining after three-dimensional printing with bio-inks of various compositions containing a C2Cl2 myoblast cell line (Gel-MA: gelatin methacrylate).
Fig. 7 shows that it was confirmed that the proliferation and differentiation of muscle cells were further improved as compared with a control group containing no tissue-derived components after printing muscle cells using a bio-ink composition containing muscle tissue-derived components (Control : Tissue-derived components-untreated control group, muscle ECM: composition group containing muscle-derived extracellular matrix).
FIG. 8 is a schematic diagram showing a method of incorporating a peptide into the bio-ink composition via chemical bonding, wherein the peptide mimics a growth factor involved in the regulation of cell differentiation.
FIG. 9 is photographic results showing a tissue-like organ prepared using the bio-ink composition of the present invention (A: liver tissue organoid, B: cardiac tissue organoid, C:
   muscle tissue organoid).
FIG. 10 is photographic results showing the preparation of a three-dimensional bio-printed and manipulated tissue structure using the bio-ink composition of the present invention (A: Ear tissue structure, B: nose tissue structure, and C: blood vessel-like tissue structure).
FIG. 11 is photographic results showing that the linkage among tissues was able to be reproduced by simultaneously printing various cells (A: hepatocyte-vascular cell printing, B: osteocyte-chondrocyte printing, C: muscle-tendon cell printing)
FIG. 12 is photographic results showing that a muscle tissue structure having an improved function was formed by simultaneously printing nerve cells and muscle cells using the composition of the present invention (red: muscle, green: nerve cell)

### MODE FOR CARRYING OUT INVENTION

Hereinafter, the present invention will be described in detail.

However, the following examples are illustrative of the present invention, and the present invention is not limited to the following examples.

### Example 1: Cell Culture

In order to evaluate the bio-ink composition of the present invention, various cells were utilized as follows: 3T3 fibroblast cell line (ATCC, Manassas VA, USA) was cultured in high glucose DMEM culture medium (Life Technologies) containing 10% fetal bovine serum (FBS, Life Technologies, Carsbad, CA, USA) and 1% penicillin/streptomycin at 37 °C in a 5% CO2 cell incubator.

Chondrocytes separated from the cartilage of a rabbit by enzymes were cultured in DMEM/F-12 culture medium (Life Techologies) containing 10% FBS and 1% penicillin/streptomycin at 37 ° C in a 5% CO2 cell incubator.

The C2C12 muscle cell line (ATCC) was cultured in high glucose DMEM culture medium containing 10% FBS and 1% penicillin/streptomycin, and then cultured in DMEM/F-12 medium and 1 % horse serum (HS, Life Technologies) for muscle fiberization.

### Example 2: Preparation of bio-ink composition

The bio-ink composition of the present invention comprises a temperature-sensitive gelatin (35 mg/mL, Sigma-Aldrich, St. Louis, Mo., USA) which has a sufficient viscosity so that printing can be performed in a uniformly mixed form of cells, hyaluronic acid (3 mg/mL, Sigma-Aldrich) which can enhance the viscosity of the bio-ink during printing and improve the uniformity of the pattern, glycerol (10 v/v %, Sigma-Aldrich) as a lubricant which reduces nozzle clogging, and a chemically modified gelatin (20 mg/mL methacrylated gelatin) which provides structural stability by cross-linking after printing. The bio-ink composition in which such components were fully mixed and dissolved was sterilized using a 0.45 um injector-type filter.

### Example 3: Evaluation of dispensing rate and structural stability of bio-ink composition

The uniformity of the pattern and the stability of the printed structure were evaluated upon printing with the bio-ink of the present invention. These are indices indicating the superiority of the bio-ink of the present invention. For evaluation, the bio-ink of the present invention was filled into a bio-printer and printed through a nozzle having a diameter of 300 um at a pressure of 50-80 kPa. The bio-printer used in this experiment is composed of a three-axis moving stage, a dispensing module capable of pneumatic injection, and an injector-type reservoir and a nozzle for charging bio-ink.

In order to evaluate the change of dispensing rate according to the concentration of gelatin or hyaluronic acid, the content of the remaining components except gelatin or hyaluronic acid was kept the same as that of Example 2. By varying the content of gelatin or hyaluronic acid, the volume of the printed bio-ink was measured over time. Using the measured values, the coefficient of variation (COV) was calculated to evaluate the printing performance according to the concentration, and the optimal content was determined based on the evaluation.

The results are shown in Table 1 below.

**[Table 1]**

| | **Conc. (mg/mL)** | **Dispensing rate (µL/sec)** | **COV (%)** |
|---|---|---|---|
| **Gelatin** | 30 | 2.73 ± 0.82 | 30.07 |
| | 35 | 0.39 ± 0.14 | 35.70 |
| | 40 | 0.28 ± 0.09 | 31.60 |
| | 45 | 0.12 ± 0.04 | 30.65 |
| **HA** | 0 | 0.40 ± 0.14 | 35.70 |
| | 3 | 0.52 ± 0.05 | 9.81 |
| | 6 | 0.45 ± 0.02 | 4.42 |

As shown in Table 1, the dispensing rate was varied depending on the gelatin concentration. Dispensing rate is the volume of bio-ink printed per hour. The lower the dispensing rate is, the higher the resolution of the pattern becomes.

In the case of gelatin, the resolution of the pattern rapidly increased at 35 mg/mL or higher. Coefficient of variation (COV) is an index representing the uniformity of the printed pattern. The higher the value of this index is, the more dispensing rate becomes uneven. As a result, the resolution of the pattern was improved at 35 mg/mL or higher of gelatin, while there was no change in uniformity.

In contrast, when the hyaluronic acid was added to the gelatin hydrogel, the dispensing rate did not change significantly (HA: hyaluronic acid). However, there was markedly improved in the uniformity of the printed pattern as shown by the low COV values. Thus, it was found that although the inclusion of HA to the bio-ink composition insignificantly contributes to the resolution of the pattern, while exerting a great influence on the uniformity of the pattern.

Therefore, the above results suggest that the inclusion of gelatin and HA in the bio-ink composition of the present invention is essential.

In addition, in order to evaluate the structural stability of the bio-ink of the present invention, the tessellated -like structure was printed three-dimensionally using the bio-ink of the present invention. The bio-printed structure was exposed to UV for 1 to 1,000 seconds for crosslinking, followed by incubation at 37 °C in a cell culture medium. The change in size was measured over time. The evaluation was carried out for 21 days.

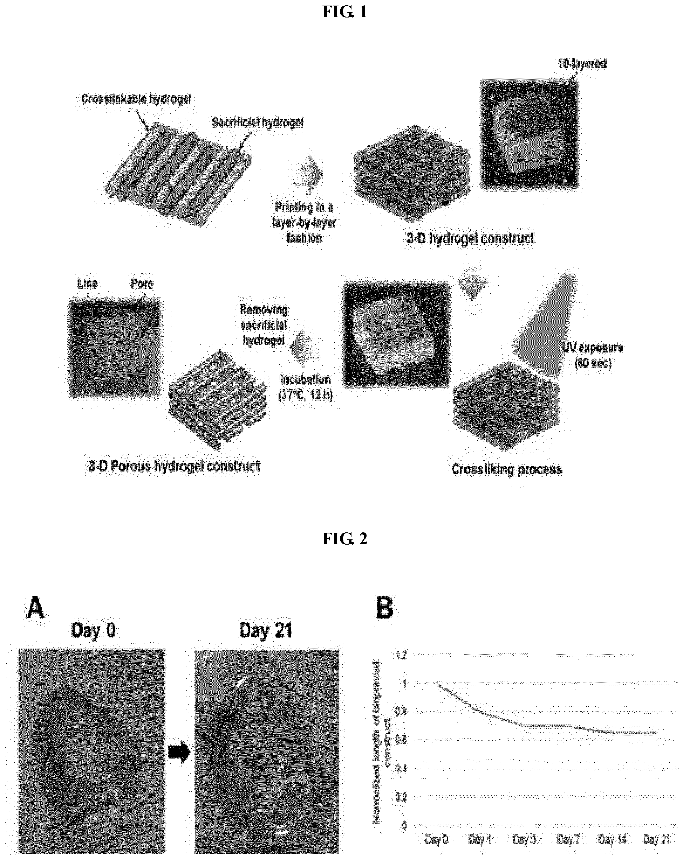

The results are shown in FIG. 2.

As shown in FIG. 2, after the bio-printed structure was cultured for 21 days, the structure stability was excellent enough to maintain about 70% of the initial size (Day 0).

Then, a change in the stability of the bio-printed structure depending on the content of the viscous enhancer contained in the bio-ink composition was determined. The bio-ink composition was prepared in the same manner as in Example 2 except that only the concentration of hyaluronic acid, which is a viscosity increasing agent, was changed to 0, 1, 2 and 3 mg/ml to evaluate the structural stability.

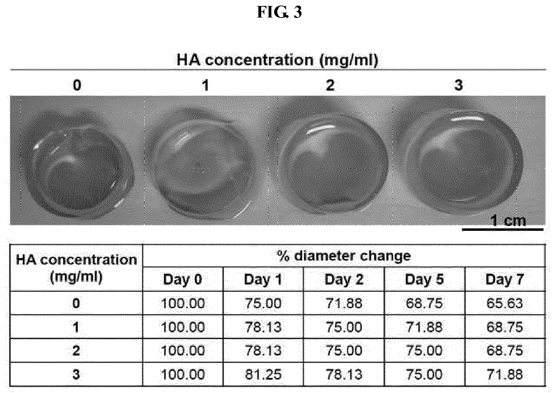

The results are shown in FIG. 3.

As shown in FIG. 3, the stability of the bio-printed structure was found to improve with the increasing content of hyaluronic acid.

### Example 4: Performance Evaluation of Bio-Ink Composition: Feed Rate and Pattern Resolution

In order to evaluate the printing performance, i.e., the resolution according to the feed rate of the bio-ink of the present invention, the bio-ink of the present invention was printed through a nozzle having a diameter of 200 um under adjustment of a feed rate of 100 to 700 mm/min.

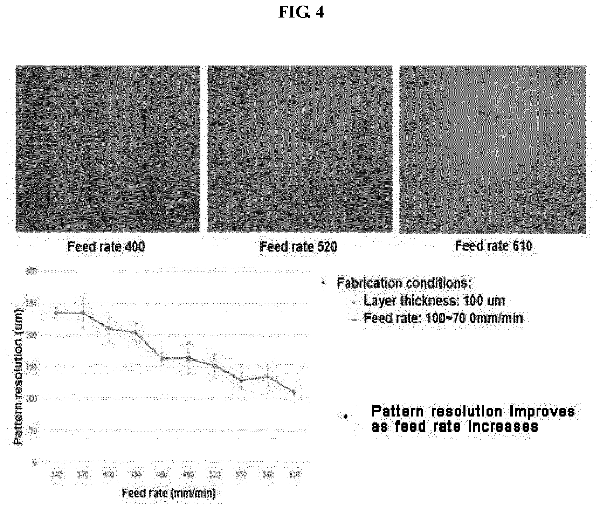

The results are shown in FIG. 4.

As shown in FIG. 4, the bio-ink of the present invention showed an improved pattern resolution as the feed rate increased, while maintaining uniformity under the feed rates as used.

### Example 5: Evaluation of cell survival rate in bio-ink composition

In order to evaluate the cell suitability of the bio-ink composition of the present invention, the bio-ink of the present invention was mixed with a 3T3 fibroblast cell line, filled in a bio printer, passed through a nozzle having a diameter of 300 um at a pressure of 50-80 kPa, completing a three-dimensional structure. As for three-dimensional structures containing bio-printed cells, cell viability was evaluated using Live/Dead assay kit (Life Technologies). The bio-ink composition used in this experiment was prepared according to the method of Example 2. By changing the molecular weight and degree of crosslinking (20, 50 and 80 %) of gelatin in gelatin methacrylate, the physical rigidity of the bio-ink was changed.

Phosphate buffer (PBS, Life Technologies) in which 0.5 uL / ml Calcein-AM and 2 uL/ mL Eth-D were dissolved was exposed to the printed bio-ink structure with the cells. Two hours later, the cells were observed under a fluorescence microscope to identify cells with green fluorescence (calcein), that is, living cells. The optimization of the bio-ink was determined under conditions of cell viability of 80% or more.

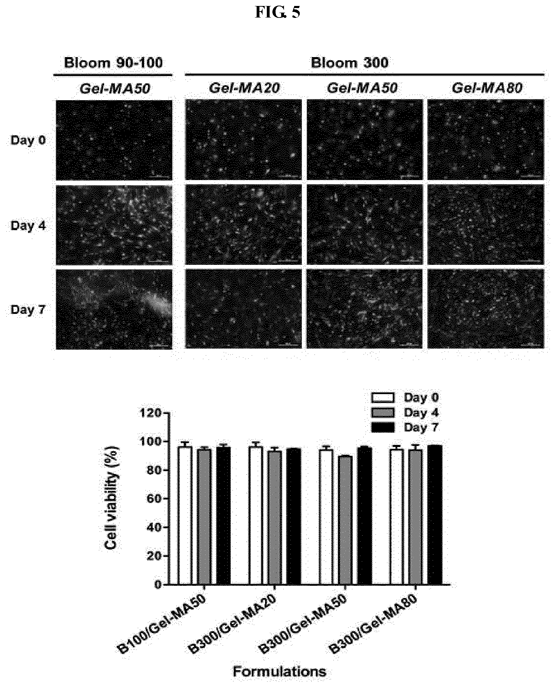

The results are shown in FIG. 5. In FIG. 5, Gel-MA 50 refers to 50% methacrylated gelatin.

As shown in FIG. 5, green fluorescence, which indicates living cells, was observed in most of the cells in the bio-printed three-dimensional structure. When quantified, the cell survival rate showed 90% or more (see the graph) under all the composition conditions used in the experiment. Thus, it was verified that the bio-ink composition of the present invention minimizes cell damage at a high shear pressure generated during printing.

The bio-ink of the same composition as the above experiment was used for printing except that the concentration of Gel-MA 50 was changed and the C2Cl2 myoblast cell line was contained. 50% methacrylated gelatin was used for the experiments with two concentrations of 20 mg/ml and 30 mg/ml, respectively. In addition, the printing conditions were as follows: feed rate of 120 mm/min and the nozzle size of 300 µTeflon nozzle.

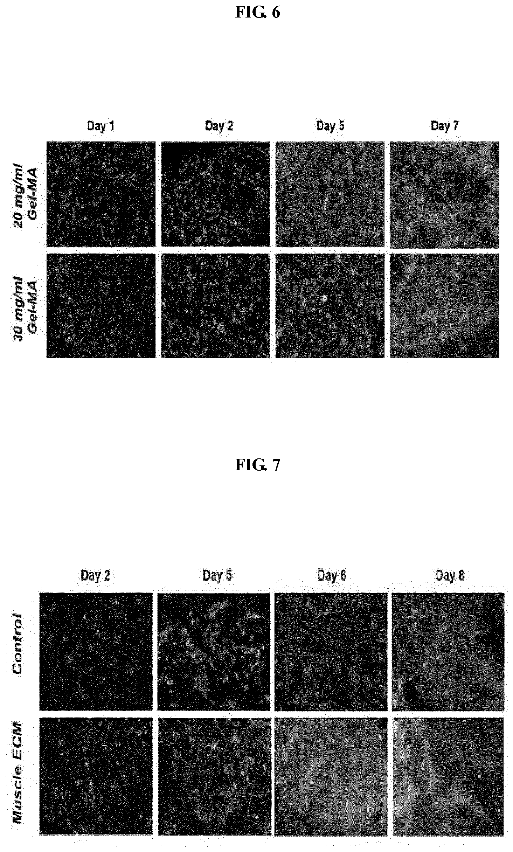

The results are shown in FIG. 6.

As shown in FIG. 6, in all the compositions, most of the cells in the bio-printed three-dimensional structure exhibited green fluorescence which indicates living cells. While the gelatin concentration is usually increased to improve the strength of the gel, such an increased gelatin concentration may reduce the diffusion rate within the gel and decrease the cell survival rate. In the case of the bio-ink composition according to the present invention, it was confirmed that Gel-MA 50 did not affect cell viability even at a concentration of 30 mg/ml.

### Example 6: Evaluation of cell proliferation and differentiation ability of bio-ink composition containing tissue-derived components

It is virtually impossible for any artificially generated composition to reproduce all of the properties possessed by natural tissue-derived components such as an extracellular matrix. Thus, by adding the de-cellularized tissue-derived components to the bio-ink compositions, it was sought to determine whether the cells could normally grow and differentiate within the bio-printed structures.

The composition contained 12% of the muscle tissue-derived component in the bio-ink composition (20 mg/ml Gel-MA 50 (bloom 300), 30 mg/ml gelatin (bloom 90-100), 3 mg/ml HA, 10% glycerol) tested above (Cell: C2C12 myoblast cell line (1 x 10⁶ cells/ml), feed rate: 120 mm/min, nozzle size: 300 µTeflon nozzle). The muscle tissue-derived components were obtained as follows: the muscle tissues of a pig were de-cellularized, followed by the separation of the remaining extracellular matrix proteins by dissolving in weak acidic solution and pepsin. Those components were neutralized and then contained in the bio-ink composition. The main components of the muscle tissue-derived components were collagen, glycosaminoglycan, growth factor and cytokine.

The results are shown in FIG. 7.

As shown in FIG. 7, when the muscle cells were printed using the bio-ink composition containing the components derived from muscle tissue, it was confirmed that the proliferation and differentiation of the cells were further improved as compared with the control group containing no tissue-derived components.

### Example 7: Preparation of bio-ink composition containing a regulator material of differentiation

The present inventors have prepared a bio-ink composition containing a regulator material of differentiation in order to induce the differentiation of stem cells (including embryonic stem cells, inducible pluripotent stem cells, and adult stem cells) contained in the bio ink composition into specific cells.

Specifically, in this Example, the bio-ink composition contained, via chemical bonding, peptides that mimic bone-forming protein-2 (BMP-2, SEQ ID NO: 1) used to induce differentiation of the bio-printed stem cells into osteocytes, growth factors such as TGF-beta3 (SEQ ID NO: 2, SEQ ID NO: 3) used to induce their differentiation into chondrocytes, or a vascular endothelial growth factor (SEQ ID NO: 4) used to induce their differentiation into vascular endothelial cells, respectively:
SEQ ID NO: 1: KIPKASSVPTELSAISTLYL
SEQ ID NO: 2: ANVAENA
SEQ ID NO: 3: LIANAK
SEQ ID NO: 4: KLTWQELYQLKYKGI

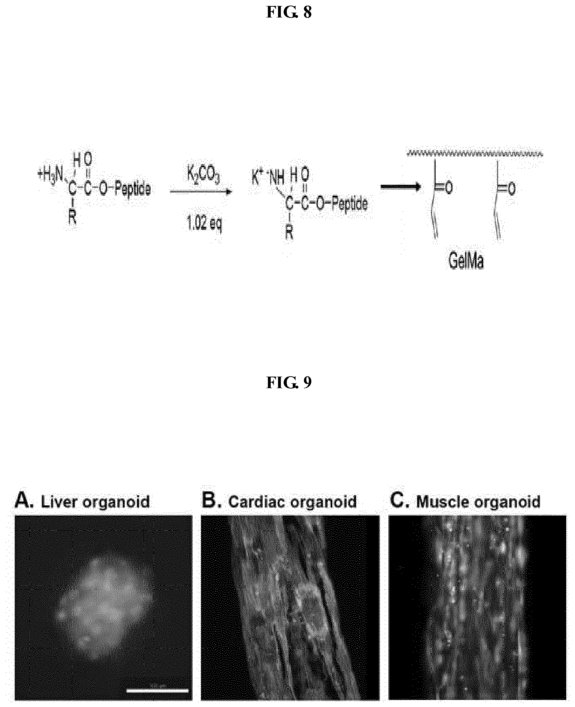

A schematic diagram of a method for preparing a bio-ink composition containing growth factors involved in the regulation of differentiation is shown in FIG. 8.

### Example 8: Preparation of tissue-like organs and implantable tissue structures using bio-ink compositions

Various tissue-like organs and implantable tissue structures were prepared using the bio-ink of the present invention. For this purpose, in order to obtain a structure that is characteristic of a specific organ, histologically stained images or bio-images (obtained by bio-imaging techniques such as CT or MRI) were converted into a 3D CAD model and then again converted into a printable motion file. The converted motion file was linked with the bio-printer to print the specific structure.

The results are shown in FIGs. 9 to 12.

As shown in FIG. 9, through observing that the liver tissue (FIG. 9A), cardiac tissue (FIG. 9B) and muscle tissue (FIG. 9C) organoids were favorably formed using the bio-ink composition of the present invention, it was confirmed that it is possible to print an internal structure with a sophisticated orientation associated with the functionality of a tissue.

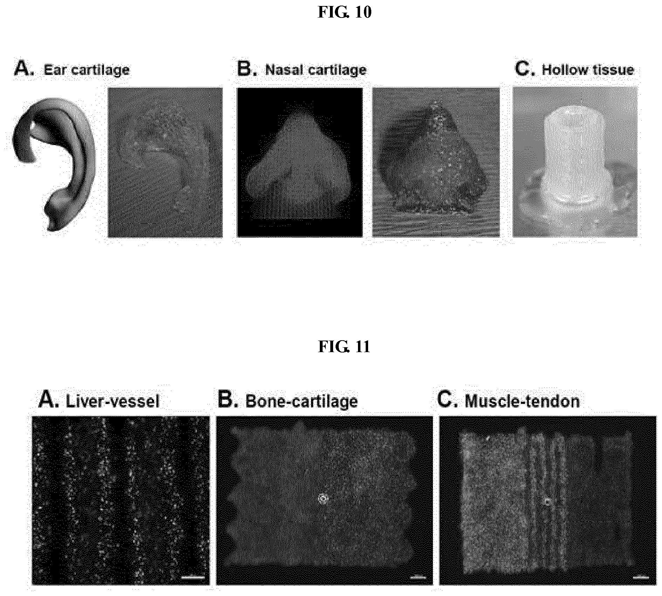

As shown in FIG. 10, it was observed that the structures of the three-dimensional bio-printed manipulated ear (FIG. 10A), nose (FIG. 10B) and tubular tissue (FIG. 10C) could also be prepared favorably, verifying that several tissues and organs that can be transplanted could be printed using the bio-ink composition developed in the present invention.

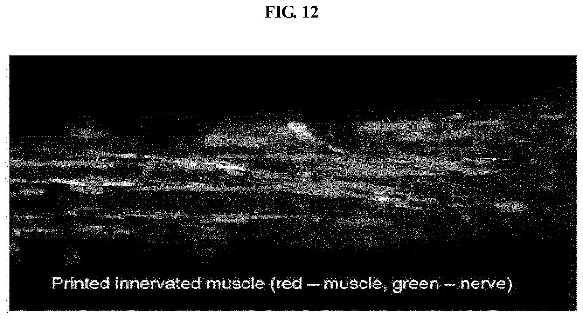

As shown in FIG. 11 and FIG. 12, it was confirmed that the interaction among tissues could be reproduced by simultaneously printing various cells using the composition of the present invention. That is, by confirming a linkage between hepatocytes and blood vessel cells (FIG. 11A), a linkage between osteocytes and chondrocytes (FIG. 11B), and a linkage between muscle cells and tendon cells (FIG. 11C), it was verified that a tissue structure improved in its functionality could be prepared using the composition of the present invention.
## Claims

1. A bio-ink composition comprising 0.05-60 x 10⁶/mL of cell, 0.1 to 10 w/v % of gelatin as a cell carrier material, 0.01 to 1 w/v % of hyaluronic acid as a viscosity enhancer, 1 to 30 v/v % of glycerol as a lubricant and 0.1 to 10 w/v % of a structural material, wherein the structural material is gelatin methacrylate.

2. The bio-ink composition of claim 1, wherein the bio-ink composition further comprises a tissue-derived component material, wherein the tissue-derived component material is a specific animal tissue which is de-cellularized and composed of an extracellular matrix being gelified.

3. The bio-ink composition of claim 1, wherein the bio-ink composition further comprises a regulator material of differentiation, which is capable of inducing the differentiation of stem cells.

4. The bio-ink composition of any of claim 1 to 3, wherein the cell is one or more selected from the group consisting of a stem cell, an osteoblast, a myoblast, a tenocyte, a neuroblast, a fibroblast, a glioblast, a germ cell, a hepatocyte, a renal cell, a sertoli cell, a chondrocyte, an epithelial cell, a cardiovascular cell, a keratinocyte, a smooth muscle cell, a cardiomyocyte, a glial cell, a endothelial cell, a hormone-secreting cell, an immune cell, a pancreatic islet cell and a neuron.

5. The bio-ink composition of any of claim 1 to 3, wherein the bio-ink composition further comprises a thrombin solution or photoinitiator.

6. A method for preparing a tissue-like organ, the method comprising:
(a) charging the bio-ink composition of any of claim 1 to 3 into a three-dimensional bio-printer;
(b) three-dimensionally printing a desired tissue-like organ; and
(c) cross-linking the three-dimensionally printed bio-ink composition.

7. The method of claim 6, wherein the method further comprises (d) culturing the tissue-like organ in a culture medium.

## Patentansprüche

1. Biotinten-Zusammensetzung, die 0,05 bis 60 x 10⁶/mL an Zellen, 0,1 bis 10 % (w/v) an Gelatine als Zellträgermaterial, 0,01 bis 1 % (w/v) an Hyaluronsäure an Viskositätsverstärker, 1 bis 30 % (v/v) an Glycerin als Gleitmittel und 0,1 bis 10 % (w/v) an einem strukturbildenden Material umfasst, wobei es sich bei dem strukturbildenden Material um Gelatinemethacrylat handelt.

2. Biotinten-Zusammensetzung nach Anspruch 1, wobei die Biotinten-Zusammensetzung weiterhin einen materiellen Bestandteil umfasst, der sich von Gewebe ableitet, wobei es sich bei dem materiellen Bestandteil, der sich von Gewebe ableitet, um ein spezifisches Tiergewebe handelt, das seiner Zellen entledigt wurde und sich aus einer gelierten extrazellulären Matrix zusammensetzt.

3. Biotinten-Zusammensetzung nach Anspruch 1, wobei die Biotinten-Zusammensetzung weiterhin ein Material zur Regulierung der Ausdifferenzierung umfasst, das dazu imstande ist, die Ausdifferenzierung von Stammzellen zu induzieren.

4. Biotinten-Zusammensetzung nach einem der Ansprüche 1 bis 3, wobei es sich bei der Zelle um eine oder mehrere handelt, die aus der Gruppe ausgewählt sind, bestehend aus einer Stammzelle, einem Osteoblasten, einem Myoblasten, einem Tenozyten, einem Neuroblasten, einem Fibroblasten, einem Glioblasten, einer Keimzelle, einem Hepatozyten, einer Nierenzelle, einer Sertoli-Zelle, einem Chondrozyten, einer Epithelzelle, einer kardiovaskulären Zellen, einem Keratinozyten, einer Zelle der glatten Muskulatur, einem Kardiomyozyten, einer Gliazelle, einer Endothelzelle, einer hormonsezernierenden Zelle, einer Immunzelle, einer Inselzelle der Bauchspeicheldrüse und einem Neuron.

5. Biotinten-Zusammensetzung nach einem der Ansprüche 1 bis 3, wobei die Biotinten-Zusammensetzung weiterhin eine Thrombinlösung oder einen Photoinitiator umfasst.

6. Verfahren zur Herstellung eines gewebeartigen Organs, wobei das Verfahren Folgendes umfasst:
(a) Befüllen eines dreidimensionalen Biodruckers mit der Biotinten-Zusammensetzung nach einem beliebigen der Ansprüche 1 bis 3;
(b) dreidimensionales Drucken eines angestrebten gewebeartigen Organs; und
(c) Quervernetzen der dreidimensional gedruckten Biotinten-Zusammensetzung.

7. Verfahren nach Anspruch 6, wobei das Verfahren weiterhin (d) das Anzüchten des gewebeartigen Organs in einem Kulturmedium umfasst.

## Revendications

1. Composition de bio-encre comprenant 0,05 à 60 x 10⁶/ml de cellules, 0,1 à 10 % en poids par unité de volume de gélatine servant de matériau de support de cellules, 0,01 à 1 % en poids par unité de volume d'acide hyaluronique servant d'agent améliorant la viscosité, 1 à 30 % en poids par unité de volume de glycérol servant de lubrifiant et 0,1 à 10 % en poids par unité de volume d'un matériau de structure, le matériau de structure étant du méthacrylate de gélatine.

2. Composition de bio-encre selon la revendication 1, ladite composition de bio-encre comprenant en outre un matériau de composant dérivé de tissu, le matériau de composant dérivé de tissu étant un tissu animal spécifique qui est décellularisé et composé d'une matrice extracellulaire étant gélifiée.

3. Composition de bio-encre selon la revendication 1, ladite composition de bio-encre comprenant en outre un matériau régulateur de différentiation, qui est capable d'induire la différentiation de cellules souches.

4. Composition de bio-encre selon une quelconque des revendications 1 à 3, dans laquelle la cellule est une ou plusieurs cellules choisies dans le groupe constitué d'une cellule souche, d'un ostéoblaste, d'un myoblaste, d'un ténocyte, d'un neuroblaste, d'un fibroblaste, d'un glioblaste, d'une cellule germinale, d'un hépatocyte, d'une cellule rénale, d'une cellule de Sertoli, d'un chondrocyte, d'une cellule épithéliale, d'une cellule cardiovasculaire, d'un kératinocyte, d'une cellule de muscle lisse, d'un cardiomyocyte, d'une cellule gliale, d'une cellule endothéliale, d'une cellule sécrétant une hormone, d'une cellule immunitaire, d'une cellule d'îlot pancréatique et d'un neurone.

5. Composition de bio-encre selon une quelconque des revendications 1 à 3, ladite composition de bio-encre comprenant en outre une solution de thrombine ou un photoinitiateur.

6. Procédé de préparation d'un organe semblable à un tissu, le procédé comprenant :
(a) le chargement de la composition de bio-encre selon une quelconque revendication 1 à 3 dans une imprimante bio à trois dimensions ;
(b) l'impression en trois dimensions d'un organe semblable à un tissu désiré ; et
(c) la réticulation de la composition de bio-encre imprimée en trois dimensions.

7. Procédé selon la revendication 6, ledit procédé comprenant en outre (d) la culture d'un organe semblable à un tissu dans un milieu de culture.
